# EUROPÄISCHE PATENTSCHRIFT

(11) **EP 2 705 902 B1**
(45) Veröffentlichungstag und Bekanntmachung des Hinweises auf die Patenterteilung: **06.05.2015**
(21) Anmeldenummer: 12006277.3
(22) Anmeldetag: 06.09.2012
(51) Int. Cl.: B01J 27/188, C07C 209/36, C07C 211/52

(54) **Verfahren zur Hydrierung von Nitroaromaten mit ausgewählten Platinkatalysatoren**
Method for hydrating nitro-aromatics with selected platinum catalysts
Procédé destiné à l'hydrogénisation de nitro-aromates avec des catalyseurs platines sélectionnés

(43) Veröffentlichungstag der Anmeldung: 12.03.2014
(73) Patentinhaber: AllessaChemie GmbH, 60386 Frankfurt am Main (DE)
(72) Erfinder: Neumann, Doris, 63069 Offenbach (DE); Ritzer, Joachim, 63817 Rodenbach (DE)
(74) Vertreter: Ackermann, Joachim

(56) Entgegenhaltungen:
- EP-A2- 0 842 920
- WO-A2-2011/036479
- DATABASE CA [Online] CHEMICAL ABSTRACTS SERVICE, COLUMBUS, OHIO, US; CHEN, ZHAOYANG ET AL: "Preparation and nitrobenzene electro- reduction performance of tungsten carbide supported platinum catalysts", XP002694921, gefunden im STN Database accession no. 2009:328967 & CHEN, ZHAOYANG ET AL: "Preparation and nitrobenzene electro- reduction performance of tungsten carbide supported platinum catalysts", HUAGONG XUEBAO (CHINESE EDITION) , 59(S1), 75-79 CODEN: HUKHAI; ISSN: 0438-1157, 2008,
- A. MALTHA ET AL: "Transition metal oxides as catalysts for the selective reduction of nitrobenzene", JOURNAL OF MOLECULAR CATALYSIS, Bd. 93, 1994, Seiten 305-316, XP002694922, Elsevier ISSN: 0304-5102

## Beschreibung

Die vorliegende Erfindung betrifft ein Verfahren zur katalytischen Hydrierung von aromatischen Nitroverbindungen zu den entsprechenden aromatischen Aminoverbindungen an durch Wolfram modifizierten Platin-Katalysatoren in Gegenwart von Phosphorverbindungen.

Die Hydrierung aromatischer Nitroverbindungen an Edelmetallkatalysatoren, wie z.B. Pd/C oder Pt/C, ist ein wichtiger Syntheseweg zu den entsprechenden aromatischen Aminoverbindungen. Problematisch bei dieser Reaktion jedoch kann deren Verlauf über eine Reihe von Zwischenstufen sein. Hydroxylamine, die als Zwischenstufen gebildet werden und bei idealem Verlauf einer Hydrierung sofort wieder abreagieren, können sich bei wenig aktiven oder ungeeigneten Katalysatoren im Reaktionsgemisch anreichern. In der Regel sind Hydroxylamine thermisch instabil. Das Reaktionsgemisch kann sich daher plötzlich exotherm zersetzen. Zudem werden häufig unerwünschte Nebenprodukte ausgehend von Hydroxylaminen gebildet. Weiterhin können bei Umsetzung mit Wasserstoff andere im Molekül befindliche Gruppen, wie z.B. Halogensubstituenten, ebenfalls hydriert bzw. abgespalten werden und sich so weitere unerwünschte Nebenprodukte bilden.

Literaturbekannt ist, dass durch Modifizierung von Edelmetallkatalysatoren mit Vanadium und Phosphorverbindungen einer Oxidationsstufe < 5 eine Anreicherung instabiler Zwischenstufen bei der Hydrierung von Nitroaromaten vermindert werden kann. So beschreiben M. Studer und P. Baumeister in WO-A-96/36597 und P. Baumeister, H.-U. Blaser und M. Studer in Catalysis Letters 1997, 49, 219-222 die Behandlung von Edelmetallkatalysatoren mit verschiedenen Metallen und sind insbesondere bei Behandlung mit Vanadiumverbindungen erfolgreich.

Einen zusätzlichen positiven Einfluss von Phosphorverbindungen einer Oxidationsstufe von < 5 beschreiben U. Siegrist, P. Baumeister, H.-U. Blaser und M. Studer in Chemical Industries (Dekker), 1998, 75 (Catalysis of Organic Reactions), 207-219 und P. Baumeister, U. Siegrist und M. Studer in EP-A-842920.

Aufdenblatten, Rhony, Belser und Quittmann beschreiben in WO-A-2011/036479 einen Prozess zur katalytischen Hydrierung von aromatischen oder heteroaromatischen Nitroverbindungen zu den korrespondierenden Aminen an Platinkatalysatoren, die mit einer Molybdän- und einer Phosphorverbindung einer Oxidationsstufe < 5 modifiziert wurden.

Zhao, Chou und Chen beschreiben in Industrial & Engineering Chemistry Research 2010, 49(4), 1669-1676 den Einsatz W-modifizierter Ni-Co-B-Katalysatoren zur Hydrierung von p-Chlornitrobenzol, setzen jedoch keine Platinkatalysatoren zur Hydrierung ein.

Becher, Birkenstock, Waldau, Witt beschreiben in DE-A-35 37 247 den Einsatz von modifizierten Raney-Ni-Katalysatoren zur Hydrierung aromatischer Dinitroverbindungen zu Diaminoverbindungen. Modifizierende Metalle hierbei sind u.a. Fe, Cr, Cu Mo, Ta, W, V, Ti, Nb, Re, Ru, Zr, Hf. Auch hier wird kein Platin eingesetzt.

Überraschend wurde nun gefunden, dass durch Wolframverbindungen in Gegenwart von Phosphorverbindungen einer Oxidationsstufe < 5 modifizierte Platinkatalysatoren vorteilhaft zur Hydrierung von gegebenenfalls substituierten Nitroaromaten eingesetzt werden können.

Aufgabe der vorliegenden Erfindung ist die Bereitstellung eines Hydrierverfahrens, das kurze Reaktionszeiten aufweist und das eine hohe Raum-Zeit-Ausbeute bietet. Das erfindungsgemäße Verfahren zeichnet sich darüber hinaus dadurch aus, dass die instabilen Zwischenstufen schnell durchlaufen werden und zu den Aminen weiterhydriert werden. Auf diesen instabilen Zwischenstufen basierende Nebenprodukte sind im Produkt nicht oder nur in Spuren nachweisbar. Des Weiteren ist z.B. die Halogenabspaltung bei der Hydrierung halogensubstituierter Nitroaromaten sehr gering.

Die vorliegende Erfindung betrifft ein Verfahren zur katalytischen Hydrierung aromatischer Nitroverbindungen mit Wasserstoff zu den korrespondierenden Aminen in Gegenwart eines geträgerten Platin enthaltenden Katalysators. Das Verfahren ist **dadurch gekennzeichnet, dass** der Platin enthaltende Katalysator mit einer Wolframverbindung und mit einer Phosphorverbindung einer Oxidationsstufe < 5 modifiziert worden ist.

Als aromatische Nitroverbindungen können im erfindungsgemäßen Verfahren beliebige carbocyclische aromatische oder heterocyclische aromatische Verbindungen eingesetzt werden, welche mindestens einen aromatischen Kern und damit kovalent verbunden mindestens eine Nitrogruppe aufweisen.

Bei den aromatischen Nitroverbindungen kann es sich um ein- oder mehrkernige carbocyclische aromatische Nitroverbindungen handeln, vorzugsweise um drei-, zwei- oder insbesondere einkerninge carbocyclische aromatische Nitroverbindungen; oder es kann sich um ein- oder mehrkernige heterocyclische aromatische Nitroverbindungen handeln, die vorzugsweise ein oder zwei Ringheteroatome ausgewählt aus der Gruppe Stickstoff, Sauerstoff und Schwefel oder Kombinationen davon aufweisen.

Diese carbocyclischen oder heterocyclischen aromatischen Verbindungen weisen mindestens eine Nitrogruppe auf, und gegebenenfalls einen oder mehrere Substituenten, beispielsweise Substituten ausgewählt aus der Gruppe der Alkyl-, Alkoxy-, Hydroxyalkyl-, Cycloalkyl-, Cycloalkoxy-, Aryl-, Aryloxy-, Aralkyl-, Aralkyloxy-, Carbonsäure-, Sulfonsäure-, Amino-, Carbonsäureester-, Carbonsäureamid-, Sulfonsäureester-, Sulfonsäureamid- und/oder Nitrilgruppen und/oder Halogenatome oder von Kombinationen von zwei oder mehreren dieser Gruppen bzw. Atome.

Vorzugsweise werden carbocyclische oder heterocyclische aromatische Verbindungen mit ein bis drei Nitrogruppen eingesetzt.

Besonders bevorzugt werden carbocyclische oder heterocyclische aromatische Verbindungen mit ein bis drei Nitrogruppen eingesetzt, die keine weiteren Substituenten aufweisen.

Ebenfalls besonders bevorzugt werden carbocyclische oder heterocyclische aromatische Verbindungen mit ein bis drei Nitrogruppen eingesetzt, die als weitere Substituenten ein bis drei Halogenatome aufweisen, insbesondere Chlor- oder Bromatome.

Beispiele für Alkylgruppen sind geradkettige oder verzweigte Alkylgruppen mit ein bis sechszehn Kohlenstoffatomen, wie Methyl, Ethyl, Propyl, Isopropyl, n-Butyl, sek.-Butyl, tert-Butyl, Pentyl, n-Hexyl, n-Heptyl, 2-Ethylhexyl, n-Octyl, n-Nonyl oder n-Decyl, n-Undecyl, n-Dodecyl, n-Tridecyl, n-Tetradecyl, n-Pentadecyl oder n-Hexadecyl.

Beispiele für Alkoxygruppen sind solche mit geradkettigen oder verzweigten Alkylgruppen mit ein bis sechszehn Kohlenstoffatomen, wie Methoxy, Ethoxy, Isopropoxy, n-Butoxy, sek.-Butoxy, tert-Butoxy, Pentyloxy, n-Hexyloxy, n-Heptyloxy, 2-Ethylhexyloxy, n-Octyloxy, n-Nonyloxy, n-Decyloxy, n-Tridecyloxy, n-Tetradecyloxy, n-Pentadecyloxy oder n- Hexadecyloxy.

Beispiele für Cycloalkylgruppen sind solche mit fünf oder sechs Ringkohlenstoffatomen, die ihrerseits wiederum substitutiert sein können, beispielsweise mit Alkylgruppen. Ein Beispiel für eine Cycloalkylgruppe ist Cyclohexyl.

Beispiele für Cycloalkoxygruppen sind solche mit fünf oder sechs Ringkohlenstoffatomen im Cycloalkylring, der seinerseits wiederum substitutiert sein kann, beispielsweise mit Alkylgruppen. Ein Beispiel für eine Cycloalkoxygruppe ist Cyclohexyloxy.

Beispiele für Arylgruppen sind solche mit sechs oder zehn Ringkohlenstoffatomen im Arylring, die ihrerseits wiederum substitutiert sein können, beispielsweise mit Alkylgruppen. Ein Beispiel für eine Arylgruppe ist Phenyl.

Ein Beispiel für eine Aralkylgruppe ist Benzyl, das seinerseits wiederum substitutiert sein kann, beispielsweise mit Alkylgruppen.

Ein Beispiel für eine Carbonsäureamidgruppe ist C₁-C₄-Acylamino, vorzugsweise Acetylamino.

Die carbocyclischen oder heterocyclischen aromatischen Nitroverbindungen können neben aromatischen Ringen noch nicht-aromatische gesättigte oder ethylenisch ungesättigte Ringe aufweisen, welche mit den aromatischen Ringen annelliert sind oder mit den aromatischen Ringen über kovalente Bindungen verknüpft sind oder mit den aromatischen Ringen ein bi- oder polycyclisches System bilden.

Beispiele für besonders bevorzugt eingesetzte aromatische Nitroverbindungen sind solche, die sich von Phenylgruppen, substitutierten Phenylgruppen, Naphthylgruppen, substituierten Naphthylgruppen, Anthracenylgruppen und substitutierten Anthracenylgruppen ableiten und die eine oder zwei Nitrogruppen und gegebenenfalls ein bis drei Chlor- oder Bromatome aufweisen.

Handelt es sich im Rahmen dieser Beschreibung um Halogen, so sind damit Fluor, Chlor, Brom oder lod gemeint. Bevorzugt werden Chlor und Brom, ganz besonders bevorzugt wird Chlor.

Besonders bevorzugt werden aromatische Nitroverbindungen der allgemeinen Strukturformel I oder II eingesetzt 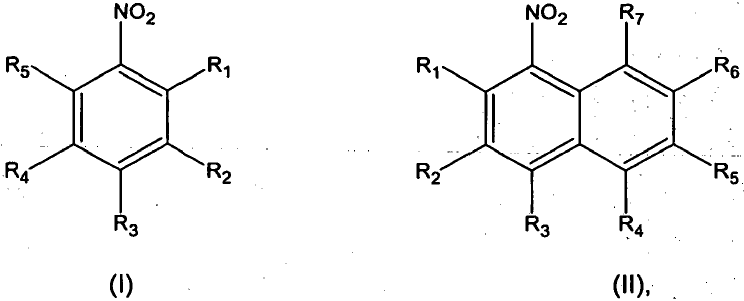 worin
R₁, R₂, R₃, R₄, R₅, R₆ und R₇ unabhängig voneinander Wasserstoff, Halogen, Alkyl, das seinerseits an einem oder mehreren C-Atomen mit Halogen- oder mit Sauerstoff oder Stickstoff enthaltenden Gruppen substituiert sein kann oder bei dem ein oder mehrere nicht benachbarte Kohlenstoffatome durch Sauerstoff-, Stickstoff- oder Schwefelatome ersetzt worden sind, oder für weitere Nitrogruppen steht, und worin gegebenenfalls ein bis drei Ringkohlenstoffatome in den Verbindungen der Formel I oder II durch Stickstoff-, Sauerstoff- und/oder Schwefelatome ersetzt sind.

Zu den Sauerstoff enthaltenden Gruppen zählen beispielsweise Alkoxy-, Cycloalkyloxy-, Aryloxy-, Carboxy- oder Carbonsäureestergruppen.

Zu den Stickstoff enthaltenden Gruppen zählen beispielsweise N-Alkylamino-, N,N-Dialkylamino, N-Cycloalkylamino-, N,N-Dicycloalkylamino, N-Arylamino-, N,N-Diarylamino-, Amino- oder Amidgruppen.

Zu den Alkylgruppen, bei denen ein oder mehrere nicht benachbarte Kohlenstoffatome durch Sauerstoff-, Stickstoff- oder Schwefelatome ersetzt worden sind, zählen beispielsweise einwertige Polyalkylenglykolreste, beispielsweise Polyethylenglykolreste, oder die entsprechenden Schwefel- oder Aminohomologen.

Die aromatischen Nitroverbindungen werden durch das erfindungsgemäße Verfahren in hoher Selektivität zu den entsprechenden aromatischen Aminen hydriert.

In den Formeln I und II stehen R₁, R₂, R₃, R₄, R₅, R₆ und R₇ unabhängig voneinander bevorzugt für Wasserstoff, C₁-C₆-Alkyl, C₁-C₆-Alkoxy, C₁-C₄-Acylamino oder für Halogen, wobei besonders bevorzugt 1 bis 4 der Reste R₁ bis R₇ von Wasserstoff verschieden sind.

Besonders bevorzugt stehen in den Verbindungen der Formel I zwei bis fünf der Substituenten R₁ bis R₅ für Wasserstoff und null bis drei der Substituenten R₁ bis R₅ für Halogen, insbesondere für Chlor oder Brom.

Besonders bevorzugt stehen in den Verbindungen der Formel I ein bis zwei der Substituenten R₁ bis R₅ für Nitro, null bis vier der Substituenten R₁ bis R₅ für Wasserstoff und null bis drei der Substituenten R₁ bis R₅ für Halogen, insbesondere für Chlor oder Brom.

Besonders bevorzugt stehen in den Verbindungen der Formel II drei bis sieben der Substituenten R₁ bis R₇ für Wasserstoff und null bis vier der Substituenten R₁ bis R₇ für Halogen, insbesondere für Chlor oder Brom.

Besonders bevorzugt stehen in den Verbindungen der Formel II ein bis zwei der Substituenten R₁ bis R₇ für Nitro, ein bis sechs der Substituenten R₁ bis R₇ für Wasserstoff und null bis vier der Substituenten R₁ bis R₇ für Halogen, insbesondere für Chlor oder Brom.

Die Hydrierungen werden in Lösung durchgeführt. Geeignete Lösungsmittel sind Wasser, Kohlenwasserstoffe, beispielsweise lineare, verzweigte oder cyclische, unsubstituierte oder mit Halogen-, Sauerstoff- oder Stickstoff substituierte C₁ - C₁₂-Alkane, sowie unsubstituierte oder mit Halogen-, Sauerstoff- oder Stickstoff substituierte ein- oder mehrkernige C₆ - C₁₆-Aryl- oder -Heteroarylverbindungen, oder Alkohole, beispielsweise lineare oder verzweigte ein - oder mehrwertige C₁-C₁₀-Alkohole, oder Ether, beispielsweise von linearen oder verzweigten ein- oder mehrwertigen C₁-C₁₀-Alkoholen abgeleitete Ether, oder Carbonsäureester, beispielsweise von linearen oder verzweigten ein - oder mehrwertigen C₁-C₁₀-Alkoholen abgeleitete Ester mit C₁ - C₄-Carbonsäuren oder Gemische der genannten Lösungsmittel bzw. der genannten Lösungsmittel mit Wasser.

Bevorzugt setzt man als Lösungsmittel Wasser, Hexan, Heptan, Octan, Cyclohexan, Toluol, o-, m- und p-Xylol, Phenol, o-, m- und p-Kresol, Anisol, Chlorbenzol, Dichlorbenzol, Methanol, Ethanol, n- oder i-Propanol, n-, sec- oder tert-Butanol, 2-Pentanol, 3-Pentanol, 2-Methylbutan-2-ol, 2-Methylpentano-2-ol, 2-Methylpentan-3-ol, 3-Methanpentan-2-ol, 4-Methylpentan-2-ol, 2,3-Dimethyl-2-butanol, 3,3-Dimethyl-2-butanol ein.

Besonders bevorzugt setzt man Heptan, Octan, Cyclohexan, Toluol, o-, m- und p-Xylol, Phenol, o-, m- und p-Kresol, Anisol, Chlorbenzol, Methanol, Ethanol, n- oder i-Propanol, n-, sec- oder tert-Butanol, 2-Pentanol, 3-Pentanol, 2-Methylbutan-2-ol, 2-Methylpentan-2-ol, 2-Methylpentan-3-ol, 3-Methylpentan-2-ol, 4-Methylpentan-2-ol, Diethylether, Diisopropylether, Diphenylether, Methylacetat, Ethylacetat, n- und i-Propylacetat, Essigsäure-n-, sec- oder tert-butylester ein.

Die Konzentration der aromatischen Nitroverbindung im Lösungsmittel wird zwischen 10- und 90%ig gewählt, bevorzugt zwischen 20- und 80%ig, besonders bevorzugt zwischen 30- und 70%ig.

Im Falle flüssiger aromatischer Nitroverbindungen können diese auch gleichzeitig als Lösungsmittel dienen.

Als Platinkatalysator lassen sich handelsübliche geträgerte Platin enthaltende Katalysatoren einsetzen. Der Platingehalt dieser Katalysatoren beträgt üblicherweise 0,3 - 10 Gew. % Platin, bevorzugt 0,3 - 8 Gew. % Platin, besonders bevorzugt 0,5 - 5 Gew. % Platin. Dabei sind die Prozentangaben auf die Trockenmasse des geträgerten Katalysators bezogen.

Als Träger kommen einen Vielzahl von Materialien in Betracht. Beispiele dafür sind Aluminiumoxid, keramische Trägermaterialien oder Kohlenstoff bzw. Graphit, beispielsweise Aktivkohle. Trägermaterialien für diese Katalysatoren sind dem Fachmann bekannt und werden in der Regel in fein verteilter Form verwendet, die ggf. zu Pellets verpresst sein können. Besonders bevorzugt wird Kohlenstoff, insbesondere Aktivkohle als Trägermaterial eingesetzt. Die Katalysatoren können als Feststoffe, z.B. als Pulver oder Pellets eingesetzt werden, aber auch als handelsübliche wasserfeuchte Paste.

Das katalytisch aktive Metall ist Platin oder eine Kombination von Platin mit anderen Metallen, beispielsweise mit Palladium oder Rhodium.

Der Katalysator kann neben dem katalytisch aktiven Metall noch mit weiteren Komponenten dotiert sein, beispielsweise mit Alkali- oder Erdalkalimetallen und/oder mit seltenen Erden.

Ganz besonders wird ein auf Kohlenstoff geträgerter Platin-Katalysator eingesetzt (nachstehend "Pt/C-Katalysator"). Es können beliebige Kohleträger eingesetzt werden. Diese Pt/C-Katalysatoren sind handelsüblich.

Die molare Menge an eingesetztem Platin, bezogen auf die Nitrokomponente, liegt in der Regel zwischen 1 * 10⁻⁷ und 1 * 10⁻² Moläquivalenten, bevorzugt zwischen 1 * 10⁻⁶ und 5 * 10⁻³ Moläquivalenten und besonders bevorzugt zwischen 5 * 10⁻⁶ und 1 * 10⁻³ Moläquivalenten.

Der Katalysator liegt im Reaktionsgemisch suspendiert vor, die aromatische Nitroverbindung wird im Lösungsmittel gelöst oder liegt bereits in flüssiger Form vor und bildet eine flüssige Phase, die mit dem in der Gasphase vorliegenden Wasserstoff reagiert.

Die aromatische Nitroverbindung kann als solche oder als Lösung auch zu dem vorgelegten Katalysator, der in der Gesamtmenge oder einem Teil des Lösungsmittels angeschlämmt ist, parallel zur Dosierung von Wasserstoff zudosiert werden.

Als geeignete Wolframverbindungen sind solche der Oxidationsstufe 4 oder 6 einzusetzen, z.B. Oxide, Halogenide oder Oxychloride, Wolframsäure oder eines deren Salze.

Bevorzugt setzt man Wolfram(IV)- oder Wolfram(VI)oxid, Wolfram(VI)oxychlorid, Wolframhexachlorid, Wolframsäure oder eines ihrer Alkali- oder Erdalkalimetallsalze, besonders bevorzugt Wolframsäure, Lithium-, Natrium, Kalium- Magnesium- oder Calciumwolframat ein.

Das molare Verhältnis Platin : Wolfram wählt man in der Regel zwischen 10 : 1 und 1 : 20, bevorzugt zwischen 15 : 1 und 1 : 15, besonders bevorzugt zwischen 8 : 1 und 1 : 8.

Die eingesetzte Phosphorverbindung einer Oxidationsstufe < 5 wählt man vorzugsweise aus der Gruppe der Phosphine PR₃₋ₙHₙ, Phosphinsäuren P(OH)R₂-ₘHₘ, Phosphinoxide P(O)R₃₋ₙHₙ, hypophosphorigen Säuren P(OH)(O)R₂₋ₘHₘ und phosphorigen Säuren P(OH)₂(O)H oder P(OH)₂(O)R, in denen R einen organischen Rest, insbesondere einen linearen oder verzweigten C₁-C₁₄-Alkylrest oder einen C₆ - C₁₆-Arylrest darstellt, n = 0 - 3 und m = 0 - 2 bedeutet, sowie aus den Salzen, Estern oder Anhydriden der erwähnten Phosphorverbindungen.

Bevorzugt wird hierfür Propanphosphonsäure, Propanphosphonsäureanhydrid, phosphorige oder hypophosphorige Säure oder ein Salz, insbesondere ein Alkali- oder Erdalkalisalz dieser Säuren eingesetzt, besonders bevorzugt phosphorige oder hypophosphorige Säure oder ein Salz, insbesondere ein Alkali- oder Erdalkalisalz dieser Säuren.

Das Mengenverhältnis der Phosphorverbindung bezogen auf die aromatische Nitroverbindung wählt man in der Regel zwischen 1 * 10⁻⁶ und 1 * 10⁻¹ Moläquivalenten, bevorzugt zwischen 1 * 10⁻⁵ und 5 * 10⁻² Moläquivalenten, besonders bevorzugt zwischen 1 * 10⁻⁴ und 1 * 10⁻³ Moläquivalenten.

Der im erfindungsgemäßen Verfahren eingesetzte Platin-Katalysator wird vor dem Einsatz mit der Wolframverbindung und mit der Phosphorverbindung, in der Regel mit einer Lösung dieser Verbindungen behandelt oder die Wolframverbindung und die Phosphorverbindung werden dem Hydriergemisch zugesetzt. In letzterem Fall ist es möglich auch vorher unbehandelte geträgerte Platin-Katalysatoren einzusetzen. Die Wolframverbindung und die Phosphorverbindung können als Substanz dem geträgerten Katalysator zugesetzt werden oder vorzugsweise als Lösung in einem geeigneten Lösungsmittel, insbesondere als wässrige Lösung. Es können auch Gemische von Wolframverbindungen und/oder von Phosphorverbindungen eingesetzt werden.

Das beschriebene Verfahren kann in beliebigen Reaktoren durchgeführt werden. Bevorzugte Beispiele dafür sind Rühr- oder Schlaufenreaktoren.

Die Hydrierzeiten des beschriebenen Verfahrens liegen im Allgemeinen zwischen ein und drei Stunden. Im Einzelfall können aber auch kürzere oder längere Reaktionszeiten verwendet werden.

Bei der Durchführung des erfindungsgemäßen Verfahrens hat sich herausgestellt, dass man gute Ausbeuten und Selektivitäten an aromatischen Aminoverbindungen im Allgemeinen bei Temperaturen von 50 bis 250°C, vorzugsweise von 60 bis 250°C, besonders bevorzugt bei 60 bis 200 °C und ganz besonders bevorzugt bei 70 bis 180°C erhält.

Das erfindungsgemäße Verfahren kann bei unterschiedlichen Wasserstoffdrucken durchgeführt werden. Die Selektivität und der Umsatz der Reaktion sind am höchsten bei einem Wasserstoffdruck von 0,5 - 60 bar. Bevorzugt hydriert man bei 0,5 - 50 bar, besonders bevorzugt bei 1 - 40 bar.

Die Vorteile des erfindungsgemäßen Verfahrens liegen in einer hohen Ausbeute und einer hohen Selektivität.

Ein weiterer Vorteil ist die Durchführbarkeit des erfindungsgemäßen Verfahrens in der Gegenwart von Wasser. Durch das Entfallen von aufwendigen Trocknungsmaßnahmen wird die Produktivität des Verfahrens erheblich gesteigert.

Ein zusätzlicher Vorteil des erfindungsgemäßen Verfahrens ist die Wiederverwendbarkeit der Katalysatoren. Dadurch können die Katalysatorkosten deutlich verringert werden.

Die folgenden Beispiele erläutern die Erfindung ohne diese zu begrenzen.

### Beispiel 1:

103 g o-Chlornitrobenzol wurden in 304 g Toluol gelöst und in einen Autoklaven gefüllt. 0,4 g eines handelsüblichen Pt/C-Katalysators (5% Pt/C, 50% wasserfeucht) wurden suspendiert in Wasser, mit 0,7 g einer 1 %igen Natriumwolframatlösung und 0,9 g 5%iger hypophosphoriger Säure versetzt und zu der Chlornitrobenzollösung in den Autoklaven gegeben. Man heizte auf 90°C und legte 20 bar Wasserstoff an. Man hydrierte, bis die Wasserstoffaufnahme abbrach.

Die Hydrierlösung enthielt 99,5% o-Chloranilin neben nur 0,04% Anilin (Enthalogenierungsprodukt).

### Beispiel 2:

Man löste 101 g p-Chlornitrobenzol in 300 g Toluol und gab die Lösung in einen Autoklaven. 0,5 g eines handelsüblichen Pt/C-Katalysators (5% Pt/C, 50% wasserfeucht) wurden suspendiert in Wasser, mit 0,8 g einer 1 %igen Natriumwolframatlösung und 0,9 g 5%iger hypophosphoriger Säure versetzt und zu der Chlornitrobenzollösung in den Autoklaven gegeben. Man heizte auf 95°C und legte 20 bar Wasserstoff an. Man hydrierte, bis die Wasserstoffaufnahme abbrach. Die Hydrierlösung enthielt 99,5% p-Chloranilin neben nur 0,01 % Anilin (Enthalogenierungsprodukt).

### Vergleichsbeispiel 1 (dotierter Katalysator ohne hypophosphorige Säure):

105 g o-Chlornitrobenzol wurden in 304 g Toluol gelöst. 0,4 g eines handelsüblichen Pt/C-Katalysators (5% Pt/C, 50% wasserfeucht) wurden suspendiert in Wasser, mit 0,65 g einer 1 %igen Natriumwolframatlösung versetzt und zusammen mit der Chlornitrobenzollösung in den Autoklaven gegeben. Bei 90 - 100°C und 20 bar Wasserstoffdruck hydrierte man, bis die Wasserstoffaufnahme abbrach. Die Hydrierlösung enthielt 81,7% o-Chloranilin neben 6,5% Anilin (Enthalogen'-ierungsprodukt) und weiteren Unbekannten.

### Vergleichsbeispiel 2 (undotierter Katalysator ohne hypophosphoriger Säure):

Man löste 101 g o-Chlornitrobenzol in 301 g Toluol. 0,4 g eines handelsüblichen Pt/C-Katalysators (5% Pt/C, 50% wasserfeucht) wurden suspendiert in Wasser und zusammen mit der Chlornitrobenzollösung in den Autoklaven gegeben. Bei 90 bis 110°C und 20 bar Wasserstoffdruck hydrierte man, bis die Wasserstoffaufnahme abbrach.

Die Hydrierlösung enthielt nur 87,6% o-Chloranilin neben 5,2% Anilin (Enthalogenierungsprodukt) und weiteren Unbekannten.

### Vergleichsbeispiel 3: (undotierter Katalysator mit hypophosphoriger Säure):

103 g o-Chlornitrobenzol wurden in 301 g Toluol gelöst und in einen Autoklaven gefüllt. 0,4 g eines handelsüblichen Pt/C-Katatysators (5% Pt/C, 50% wasserfeucht) wurden suspendiert in Wasser, mit 0,9 g 5%iger hypophosphoriger Säure versetzt und zu der Chlornitrobenzollösung in den Autoklaven gegeben. Man heizte auf 90°C und legte 20 bar Wasserstoff an. Die Wasserstoffaufnahme brach ab, bevor die theoretisch berechnete Menge aufgenommen worden war. Die Analyse der Reaktionslösung zeigte, dass neben nicht hydriertem Edukt und o-Chloranilin eine Reihe von Unbekannten vorlag.

## Patentansprüche

1. Verfahren zur katalytischen Hydrierung aromatischer Nitroverbindungen mit Wasserstoff zu den korrespondierenden Aminen in Gegenwart eines geträgerten Platin enthaltenden Katalysators, **dadurch gekennzeichnet, dass** der Platin enthaltende Katalysator mit einer Wolframverbindung und mit einer Phosphorverbindung einer Oxidationsstufe < 5 modifiziert worden ist.

2. Verfahren nach Anspruch 1, **dadurch gekennzeichnet, dass** die aromatische Nitroverbindung eine carbocyclisch-aromatische oder heterocyclischaromatische ein- oder mehrkernige Verbindung ist, die mit mindestens einer Nitrogruppe substituiert ist und die gegebenenfalls noch weitere Substituenten aufweisen kann, vorzugsweise Halogenatome.

3. Verfahren nach Anspruch 2, **dadurch gekennzeichnet, dass** die aromatische Nitroverbindung eine Verbindung der allgemeinen Strukturformel I oder II ist 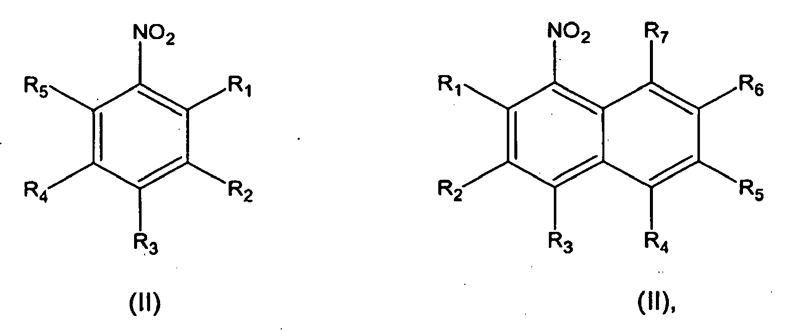 worin
R₁, R₂, R₃, R₄, R₅, R₆ und R₇ unabhängig voneinander Wasserstoff, Halogen, Alkyl, das seinerseits an einem oder mehreren C-Atomen mit Halogen- oder mit Sauerstoff oder Stickstoff enthaltenden Gruppen substituiert sein kann oder bei dem ein oder mehrere nicht benachbarte Kohlenstoffatome durch Sauerstoff-, Stickstoff- oder Schwefelatome ersetzt worden sind, oder für weitere Nitrogruppen steht, und
worin gegebenenfalls ein bis drei Ringkohlenstoffatome in den Verbindungen der Formel I oder II durch Stickstoff- , Sauerstoff- und/oder Schwefelatome ersetzt sind.

4. Verfahren nach einem der Ansprüche 1 bis 3, **dadurch gekennzeichnet, dass** die Hydrierung in Lösung durchgeführt wird.

5. Verfahren nach Anspruch 4, **dadurch gekennzeichnet, dass** als Lösungsmittel Wasser, lineare, verzweigte oder cyclische, unsubstituierte oder mit Halogenatomen oder mit Sauerstoff oder Stickstoff enthaltenden Gruppensubstituierte C₁ bis C₁₂-Alkane, unsubstituierte oder mit Halogenatomen oder mit Sauerstoff oder mit Stickstoff enthaltenden Gruppen substituierte ein- oder mehrkernige C₆ bis C₁₆-Aryl- oder Heteroarylverbindungen oder ein linearer oder verzweigter ein- oder mehrwertiger C₁ bis C₁₀-Alkohol oder Gemische der genannten Lösungsmittel oder Gemische der genannten Lösungsmittel mit Wasser eingesetzt werden.

6. Verfahren nach Anspruch 5, **dadurch gekennzeichnet dass** als Lösungsmittel Hexan, Heptan, Octan, Cyclohexan, Toluol, o-, m- und p-Xylol, Phenol, o-, mund p-Kresol, Anisol, Chlorbenzol, Dichlorbenzol, Methanol, Ethanol, n- oder i-Propanol, n-, sec- oder tert-Butanol, 2-Pentanol, 3-Pentanol, 2-Methylbutan-2-ol, 2-Methylpentano-2-ol, 2-Methylpentan-3-ol, 3-Methylpentan-2-ol, 4-Methyl-pentan-2-ol, 2,3-Dimethyl-2-butanol, 3,3-Dimethyl-2-butanol eingesetzt werden.

7. Verfahren nach einem der Ansprüche 1 bis 6, **dadurch gekennzeichnet, dass** die Konzentration der aromatischen Nitroverbindung im Lösungsmittel 10 bis 90 Gew. %, bevorzugt 20 - 80 Gew. %, besonders bevorzugt 30 - 70 Gew. % beträgt.

8. Verfahren nach einem der Ansprüche 1 bis 7, **dadurch gekennzeichnet, dass** der Platin enthaltende Katalysator einen Platingehalt von 0,3 - 10 Gew. % Platin aufweist, bevorzugt 0,3 - 8 Gew. % Platin, besonders bevorzugt 0,5 - 5 Gew. % Platin.

9. Verfahren nach einem der Ansprüche 1 bis 8, **dadurch gekennzeichnet, dass** als Katalysatorträger Aluminiumoxid, keramische Trägermaterialien oder Aktivkohle zum Einsatz kommen.

10. Verfahren nach einem der Ansprüche 1 bis 9, **dadurch gekennzeichnet, dass** die Katalysatoren als Feststoffe, insbesondere als Pulver oder Pellets oder als wasserfeuchte Paste eingesetzt werden.

11. Verfahren nach einem der Ansprüche 1 bis 10, **dadurch gekennzeichnet, dass** die molare Menge an eingesetztem Platin bezogen auf die Nitrokomponente zwischen 1 x 10⁻⁷ und 1 x 10⁻² Moläquivalenten, bevorzugt zwischen 1 x 10⁻⁶ und 5 x10⁻³ Moläquivalenten und besonders bevorzugt zwischen 5 x 10⁻⁶ und 1 x 10⁻³ Moläquivalenten liegt.

12. Verfahren nach einem der Ansprüche 1 bis 11, **dadurch gekennzeichnet, dass** als Wolframverbindung eine Wolframverbindung der Oxidationsstufe 4 oder 6 eingesetzt wird, insbesondere ein Oxid, Halogenid oder Oxychlorid, Wolframsäure oder eines deren Salze.

13. Verfahren nach Anspruch 12, **dadurch gekennzeichnet, dass** als Wolframverbindung Wolfram(IV)- oder Wolfram(VI)oxid, Wolfram(VI)oxychlorid, Wolframhexachlorid, Wolframsäure oder eines ihrer Alkali- oder Erdalkalimetallsalze, besonders bevorzugt Wolframsäure, Lithium-, Natrium, Kalium- Magnesium- oder Calciumwolframat eingesetzt werden.

14. Verfahren nach einem der Ansprüche 1 bis 13, **dadurch gekennzeichnet, dass** das molare Verhältnis Platin : Wolfram 5 : 1 bis 1 : 5, bevorzugt 4 : 1 bis 1 : 4, besonders bevorzugt 3 : 1 bis 1 : 3 beträgt.

15. Verfahren nach einem der Ansprüche 1 bis 14, **dadurch gekennzeichnet, dass** die eingesetzte Phosphorverbindung einer Oxidationsstufe < 5 aus der Gruppe der Phosphine PR₃₋ₙHₙ, der Phosphinsäuren P(OH)R₂₋ₘHₘ, der Phosphinoxide P(O)R₃₋ₙHₙ, der hypophosphorigen Säuren P(OH)(O)R₂₋ₘHₘ und der phosphorigen Säuren P(OH)₂(O)H oder P(OH)₂(O)R ausgewählt wird, in denen R einen linearen oder verzweigten C₁-C₁₄-Alkylrest oder einen C₆-C₁₆-Arylrest darstellt, n = 0 - 3, m = 0 - 2, sowie aus den Salzen, Anhydriden oder Estern der erwähnten-Phosphorverbindungen-stammt.

16. Verfahren nach Anspruch 15, **dadurch gekennzeichnet, dass** als Phosphorverbindung Propanphosphonsäure, phosphorige oder hypophosphorige Säure oder ein Salz, Anhydrid oder Ester dieser Säuren eingesetzt wird.

17. Verfahren nach einem der Ansprüche 1 bis 16, **dadurch gekennzeichnet, dass** das Mengenverhältnis der Phosphorverbindung bezogen auf die aromatische Nitroverbindung zwischen 1 x 10⁻⁶ und 1 x 10⁻¹ Moläquivalenten, bevorzugt zwischen 1 x 10⁻⁵ und 5 x 10⁻² Moläquivalenten, besonders bevorzugt zwischen 1 x 10⁻⁴ und 1 x 10⁻³ Moläquivalenten liegt.

## Claims

1. Method for the catalytic hydrogenation of aromatic nitro compounds with hydrogen to the corresponding amines in the presence of a supported catalyst comprising platinum, **characterized in that** the catalyst comprising platinum has been modified with a tungsten compound and with a phosphorus compound in an oxidation state of < 5.

2. Method according to Claim 1, **characterized in that** the aromatic nitro compound is a carbocyclic aromatic or heterocyclic aromatic, monocyclic or polycyclic compound which is substituted with at least one nitro group and which optionally may have further substituents, preferably halogen atoms.

3. Method according to Claim 2, **characterized in that** the aromatic nitro compound is a compound of the general structural formula I or II 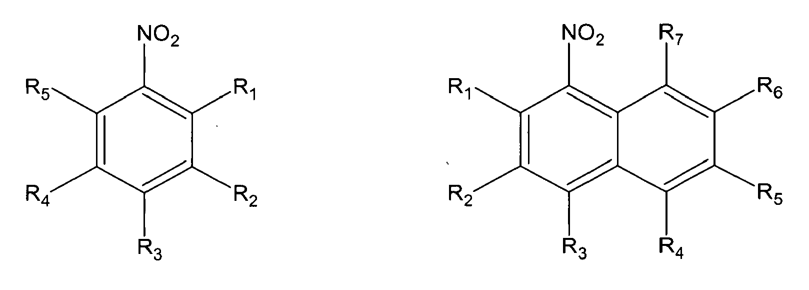 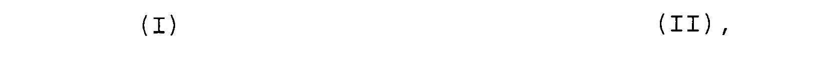 where
R₁, R₂, R₃, R₄, R₅, R₆ and R₇ are each independently hydrogen, halogen, alkyl, which may in turn be substituted at one or more carbon atoms with halogen groups or with groups containing oxygen or nitrogen, or in which one or more nonadjacent carbon atoms have been replaced by oxygen, nitrogen or sulfur atoms, or are further nitro groups, and
where optionally one to three ring carbon atoms in the compounds of the formula I or II are replaced by nitrogen, oxygen and/or sulfur atoms.

4. Method according to any of Claims 1 to 3, **characterized in that** the hydrogenation is carried out in solution.

5. Method according to Claim 4, **characterized in that** the solvents used are water, linear, branched or cyclic C₁ to C₁₂ alkanes, unsubstituted or substituted with halogen atoms or with groups containing oxygen or nitrogen, monocyclic or polycyclic C₆ to C₁₆ aryl or heteroaryl compounds, unsubstituted or substituted with halogen atoms or with groups containing oxygen or nitrogen, or a linear or branched monohydric or polyhydric C₁ to C₁₀ alcohol or mixtures of said solvents or mixtures of said solvents with water.

6. Method according to Claim 5, **characterized in that** the solvents used are hexane, heptane, octane, cyclohexane, toluene, *o*-, *m*- and *p*-xylene, phenol, *o*-, *m*- and *p*-cresol, anisole, chlorobenzene, dichlorobenzene, methanol, ethanol, *n*- or isopropanol, *n*-, *sec-* or *tert*-butanol, 2-pentanol, 3-pentanol, 2-methylbutan-2-ol, 2-methylpentan-2-ol, 2-methylpentan-3-ol, 3-methylpentan-2-ol, 4-methyl-pentan-2-ol, 2,3-dimethyl-2-butanol, 3,3-dimethyl-2-butanol.

7. Method according to any of Claims 1 to 6, **characterized in that** the concentration of the aromatic nitro compound in the solvent is 10 to 90% by weight, preferably 20 - 80% by weight, particularly preferably 30 - 70% by weight.

8. Method according to any of Claims 1 to 7, **characterized in that** the catalyst comprising platinum has a platinum content of 0.3 - 10% by weight of platinum, preferably 0 . 3 - 8% by weight of platinum, particularly preferably 0.5 - 5% by weight of platinum.

9. Method according to any of Claims 1 to 8, **characterized in that** the catalyst supports used are aluminum oxide, ceramic support materials or activated charcoal.

10. Method according to any of Claims 1 to 9, **characterized in that** the catalysts used are solids, particularly powders or pellets, or water-moist pastes.

11. Method according to any of Claims 1 to 10, **characterized in that** the molar amount of platinum used, based on the nitro component, is between 1 x 10⁻⁷ and 1 x 10⁻² mole equivalents, preferably between 1 x 10⁻⁶ and 5 x 10⁻³ mole equivalents and particularly preferably between 5 x 10⁻⁶ and 1 x 10⁻³ mole equivalents.

12. Method according to any of Claims 1 to 11, **characterized in that** the tungsten compound used is a tungsten compound in an oxidation state of 4 or 6, particularly an oxide, halide or oxychloride, tungstic acid or a salt thereof.

13. Method according to Claim 12, **characterized in that** the tungsten compounds used are tungsten(IV) or tungsten(VI) oxide, tungsten(VI) oxychloride, tungsten hexachloride, tungstic acid or an alkali metal or alkaline earth metal salt thereof, particularly preferably tungstic acid or tungstates of lithium, sodium, potassium, magnesium or calcium.

14. Method according to any of Claims 1 to 13, **characterized in that** the molar ratio of platinum : tungsten is from 5 : 1 to 1 : 5, preferably from 4 : 1 to 1 : 4, especially preferably from 3 : 1 to 1 : 3.

15. Method according to any of Claims 1 to 14, **characterized in that** the phosphorus compound used in an oxidation state of < 5 is selected from the group consisting of the phosphines PR₃₋ₙHₙ, the phosphinic acids P(OH)R₂₋ₘHₘ, the phosphine oxides P(O)R₃₋ₙHₙ, the hypophosphorous acids P(OH)(O)R₂₋ₘHₙ and the phosphorous acids P(OH)₂(O)H or P(OH)₂(O)R, in which R is a linear or branched C₁-C₁₄ alkyl residue or a C₆ - C₁₆ aryl residue, n = 0 - 3, m = 0 - 2, and is also derived from the salts, anhydrides or esters of the phosphorus compounds mentioned.

16. Method according to Claim 15, **characterized in that** the phosphorus compound used is propanephosphonic acid, phosphorous or hypophosphorous acid or a salt, anhydride or ester of these acids.

17. Method according to any of Claims 1 to 16, **characterized in that** the proportion of the phosphorus compound, based on the aromatic nitro compound, is between 1 x 10⁻⁶ and 1 x 10⁻¹ mole equivalents, preferably between 1 x 10⁻⁵ and 5 x 10⁻² mole equivalents, particularly preferably between 1 x 10⁻⁴ and 1 x 10⁻³ mole equivalents.

## Revendications

1. Procédé d'hydrogénation catalytique de composés nitrés aromatiques avec de l'hydrogène en les amines correspondantes en présence d'un catalyseur contenant du platine sur substrat, **caractérisé en ce que** le catalyseur contenant du platine a été modifié avec un composé de tungstène et avec un composé de phosphore d'un état d'oxydation < 5.

2. Procédé selon la revendication 1, **caractérisé en ce que** le composé nitré aromatique est un composé carbocyclique aromatique ou hétérocyclique aromatique mono- ou polycyclique qui est substitué par au moins un groupe nitré et qui peut éventuellement présenter encore d'autres substituants, de préférence des atomes d'halogène.

3. Procédé selon la revendication 2, **caractérisé en ce que** le composé nitré aromatique est un composé de la formule structurelle générale I ou II 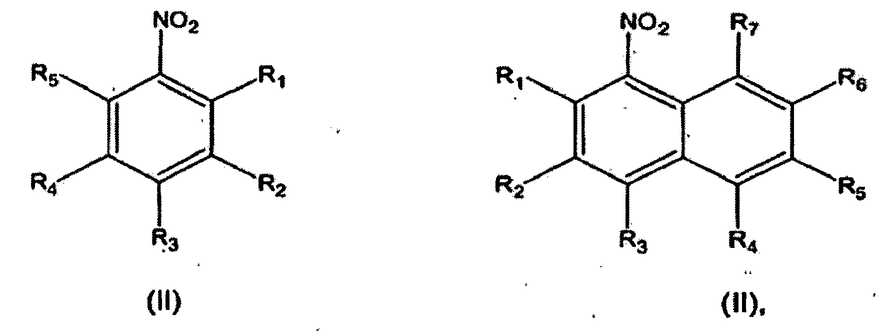 dans laquelle
R₁, R₂, R₃, R₄, R₅, R₆ et R₇ représentent indépendamment les uns des autres l'hydrogène, un halogène, un alkyle qui peut être substitué de son côté sur un ou plusieurs atomes de C par des groupes halogène ou par des groupes contenant de l'oxygène ou de l'azote ou pour lequel un ou plusieurs atomes de carbone non voisins peuvent être remplacés par des atomes d'oxygène, d'azote ou de soufre, ou représentent d'autres groupes nitrés, et
dans laquelle un à trois atomes de carbone cycliques sont éventuellement remplacés dans les composés de la formule I ou II par des atomes d'azote, d'oxygène et/ou de soufre.

4. Procédé selon une des revendications 1 à 3, **caractérisé en ce que** l'hydrogénation est effectuée en solution.

5. Procédé selon la revendication 4, **caractérisé en ce que** de l'eau, des alcanes en C₁ à C₁₂ linéaires, ramifiés ou cycliques, non substitués ou substitués par des atomes d'halogène ou par des groupes contenant de l'oxygène ou de l'azote, des composés aryle ou hétéroaryle en C₆ à C₁₆ mono- ou polycycliques non substitués ou substitués par des atomes d'halogène ou par des groupes contenant de l'oxygène ou de l'azote ou un alcool en C₁ à C₁₀ mono- ou polyvalent linéaire ou ramifié ou des mélanges des solvants cités ou des mélanges des solvants cités avec de l'eau sont utilisés comme solvant.

6. Procédé selon la revendication 5, **caractérisé en ce que** de l'hexane, heptane, octane, cyclohexane, toluène, o-, m- et p-xylène, phénol, o-, m- et p-crésol, anisol, chlorobenzène, dichlorobenzène, méthanol, éthanol, n- ou i-propanol, n-, sec- ou tert-butanol, 2-pentanol, 3-pentanol, 2-méthylbutan-2-ol, 2-méthylpentan-2-ol, 2-méthylpentan-3-ol, 3-méthylpentan-2-ol, 4-méthylpentan-2-ol, 2,3-diméthyl-2-butanol, 3,3-diméthyl-2-butanol sont utilisés comme solvant.

7. Procédé selon une des revendications 1 à 6, **caractérisé en ce que** la concentration du composé nitré aromatique dans le solvant se monte à 10 jusqu'à 90 % en poids, de préférence 20 à 80 % en poids, de manière particulièrement préférée 30 à 70 % en poids.

8. Procédé selon une des revendications 1 à 7, **caractérisé en ce que** le catalyseur contenant du platine présente une teneur en platine de 0,3 à 10 % en poids de platine, de préférence 0,3 à 8 % en poids de platine, de manière particulièrement préférée 0,5 à 5 % en poids de platine.

9. Procédé selon une des revendications 1 à 8, **caractérisé en ce que** de l'oxyde d'aluminium, des matériaux supports céramiques ou du charbon actif sont utilisés comme support de catalyseur.

10. Procédé selon une des revendications 1 à 9, **caractérisé en ce que** les catalyseurs sont utilisés comme solides, notamment comme poudre ou granulés ou comme pâte humide.

11. Procédé selon une des revendications 1 à 10, **caractérisé en ce que** la quantité molaire en platine utilisé par rapport au composant nitré se situe entre 1 x 10⁻⁷ et 1 x 10⁻² équivalents molaires, de préférence entre 1 x 10⁻⁶ et 5 x 10⁻³ équivalents molaires et de manière particulièrement préférée entre 5 x 10⁻⁶ et 1 x 10⁻³ équivalents molaires.

12. Procédé selon une des revendications 1 à 11, **caractérisé en ce qu'**un composé de tungstène de l'état d'oxydation 4 ou 6 est utilisé comme composé de tungstène, notamment un oxyde, halogénure ou oxychlorure, de l'acide tungstique ou un de ses sels.

13. Procédé selon la revendication 12, **caractérisé en ce que** de l'oxyde de tungstène(IV) ou tungstène(VI), de l'oxychlorure de tungstène(VI), de l'hexachlorure de tungstène, de l'acide tungstique ou un de ses sels alcalins ou alcalinoterreux, de manière particulièrement préférée l'acide tungstique, le tungstate de lithium, sodium, potassium, magnésium ou calcium, sont utilisés comme composé de tungstène.

14. Procédé selon une des revendications 1 à 13, **caractérisé en ce que** le rapport molaire platine/tungstène se monte à 5/1 à 1/5, de préférence 4/1 à 1/4, de manière particulièrement préférée 3/1 à 1/3.

15. Procédé selon une des revendications 1 à 14, **caractérisé en ce que** le composé de phosphore utilisé d'un état d'oxydation < 5 est sélectionné parmi le groupe des phosphines PR₃₋ₙHₙ, des acides phosphiniques P(OH)R₂₋ₘHₘ, des oxydes phosphiniques P(O)R₃₋ₙHₙ, des acides hypophosphoriques P(OH)(O)R₂₋ₘHₘ et des acides phosphoriques P(OH)₂(O)H ou P(OH)₂(O)R dans lesquels R représente un radical C₁-C₁₄-alkyle linéaire ou ramifié ou un radical C₆-C₁₆-aryle, n = 0 à 3, m = 0 à 2, et provient des sels, anhydrides ou esters des composés de phosphore mentionnés.

16. Procédé selon la revendication 15, **caractérisé en ce que** de l'acide propanephosphonique, de l'acide phosphorique ou hypophosphorique ou un sel, anhydride ou ester de ces acides est utilisé comme composé de phosphore.

17. Procédé selon une des revendications 1 à 16, **caractérisé en ce que** le rapport quantitatif du composé de phosphore par rapport au composé nitré aromatique se situe entre 1 x 10⁻⁶ et 1 x 10⁻¹ équivalents molaires, de préférence entre 1 x 10⁻⁵ et 5 x 10⁻² équivalents molaires, de manière particulièrement préférée entre 1 x 10⁻⁴ et 1 x 10⁻³ équivalents molaires.
